# EUROPEAN PATENT APPLICATION

(11) **EP 3 594 816 A1**
(43) Date of publication of application: **15.01.2020**
(21) Application number: 18763838.2
(22) Date of filing: 02.03.2018
(51) Int. Cl.: G06F 11/30

(54) **DATA PROCESSING DEVICE AND DATA VOLUME REDUCTION METHOD**

(30) Priority: 09.03.2017 CN 201710136368
(71) Applicant: Qingyuan Global Technology Services Ltd., Qingyuan City, Guangdong 51180 (CN)
(72) Inventor: LUH, Yih Ping, Taipei 104 (TW)
(74) Representative: dompatent von Kreisler Selting Werner - Partnerschaft von Patent- und Rechtsanwälten mbB
(86) International application number: PCT/CN2018/077912
(87) International publication number: WO 2018/161861

(57) **Abstract**

A data processing apparatus and a data amount reducing method are disclosed. The data processing apparatus includes a receiving unit and a numerical transforming unit. The receiving unit is used to receive M original detection data, wherein M is a positive integer. The numerical transforming unit is coupled to the receiving unit and used to perform a numerical transforming process on the M original detection data to generate K numerical transformed data, wherein K is a positive integer smaller than M. Therefore, the amount of the K numerical transformed data can be smaller than the amount of the M original detection data to achieve the effect of reducing the amount of data.

## Description

### BACKGROUND OF THE INVENTION

### 1. Field of the invention

The invention relates to reduce an amount of data; in particular, to a data processing apparatus and a data amount reducing method.

### 2. Description of the prior art

With the continuous evolution of data processing technology, in order to be applicable to various portable devices and wearable devices, the volume of data processing devices (such as microprocessors or computing chips) is designed to be smaller and smaller. There is also a limitation to the amount of data that can be stored and processed.

Since many portable devices and wearable devices provide various detection functions, in order to achieve more accurate detection results, portable devices and wearable devices are often provided with considerable detection. The unit separately detects and transmits the detected original detection data to a data processing device (such as a microprocessor or an arithmetic chip).

However, since the number of detection units is too large and each detected unit has detected data to be transmitted to the data processing device, the amount of data received by the data processing device is quite large, far exceeding the upper limit of the data processing device can be stored and processed, so that the data processing device fails to complete the storage and data processing procedures.

### SUMMARY OF THE INVENTION

Therefore, the invention provides a data processing apparatus and a data amount reducing method to solve the above-mentioned problems of the prior arts.

A preferred embodiment of the invention is a data processing apparatus. In this embodiment, the data processing apparatus includes a receiving unit and a numerical transforming unit. The receiving unit is used to receive M original detection data, wherein M is a positive integer. The numerical transforming unit is coupled to the receiving unit and used to perform a numerical transforming process on the M original detection data to generate K numerical transformed data, wherein K is a positive integer smaller than M. Therefore, the amount of the K numerical transformed data can be smaller than the amount of the M original detection data to achieve the effect of reducing the amount of data.

In an embodiment, the numerical transforming unit uses a numerical transforming mechanism to perform the numerical transforming process on the M original detection data.

In an embodiment, the numerical transforming mechanism is a Fourier transform mechanism or a Laplace transform mechanism.

In an embodiment, the data processing apparatus is a Bluetooth chip or an Internet of Thing (IoT) chip and can be connected to a cloud database or a mobile communication device through a network.

In an embodiment, the numerical transforming unit further compares the M original detection data with a preset value, and generates the K numerically transformed data according to the K original detection data that are greater than the preset value among the M original detection data.

In an embodiment, the M original detection data are M capacitance variations sensed by the M capacitive sensing nodes of a capacitive pressure detection insole, and the M capacitance variations respectively correspond to M detecting positions of a foot.

In an embodiment, one of the M capacitive sensing nodes of the capacitive pressure detection insole is disposed at an intersection of a capacitive yarn and a conductive yarn, and two electrically conductive coatings are formed on a surface of the capacitive yarn.

Another preferred embodiment of the invention is a data amount reducing method. In this embodiment, the data amount reducing method includes steps of: (a) providing M original detection data, wherein M is a positive integer; and (b) performing a numerical transforming process on the M original detection data to generate K numerical transformed data, wherein K is a positive integer smaller than M, so that an amount of the K numerical transformed data can be smaller than an amount of the M original detection data.

Compared to the prior art, the data processing apparatus and the data amount reducing method of the invention use the numerical transforming mechanism such as Fourier transform or Laplace transform or the screening mechanism comparing with a preset value to process the huge amount of received data to significantly reduce the amount of data, so that the data processing apparatus can store and process the numerical transformed data in real time, thus effectively avoiding the disadvantages of the prior art that the amount of data is too huge to be stored and processed.

The advantage and spirit of the invention may be understood by the following detailed descriptions together with the appended drawings.

### BRIEF DESCRIPTION OF THE APPENDED DRAWINGS

FIG. 1 illustrates a schematic diagram showing that the capacitive pressure detection insole 2 is disposed in the shoe 1 of the invention.
FIG. 2 illustrates a schematic diagram showing that the capacitive pressure detection insole 2 has capacitive sensing nodes N in the invention.
FIG. 3 illustrates a schematic diagram showing that the capacitive sensing nodes N in the capacitive pressure detection insole 2 is disposed under the thermoplastic polyester elastomer (TPEE) layer TP and the operating chip CH is embedded in the capacitive pressure detection insole 2 in the invention.
FIG. 4 illustrates a schematic diagram showing that when the capacitive pressure detection insole 2 is subjected to the pressure applied by the user's foot FT, the operating chip CH receives the capacitance variations corresponding to the detection positions P1, P2, P3, ... of the foot FT sensed by the capacitive sensing nodes N1, N2, N3, ... respectively and connects to the cloud database DB or the mobile communication device MB through the network NET.
FIG. 5 illustrates a schematic diagram showing that the capacitive yarn L1 and the conductive yarn L2 are twisted.
FIG. 6 illustrates a functional block diagram of the data processing apparatus of the invention.
FIG. 7 illustrates a flow chart showing that the method for reducing the amount of data applied to the capacitive pressure detection insole of the invention.

### DETAILED DESCRIPTION OF THE INVENTION

A preferred embodiment of the invention is a data processing apparatus. In this embodiment, the data processing apparatus can be a microprocessor, a Bluetooth chip or an Internet of Thing (IoT) chip, and can be embedded in a capacitive pressure detection insole, but not limited to this. When the data processing apparatus receives M original detection data, the data processing apparatus will perform a numerical transforming process on the M original detection data to generate K numerically transformed data, wherein M and K are both positive integers and K is smaller than M, the amount of the K numerically transformed data will be less than the amount of the M original detection data, so as to reduce the amount of data.

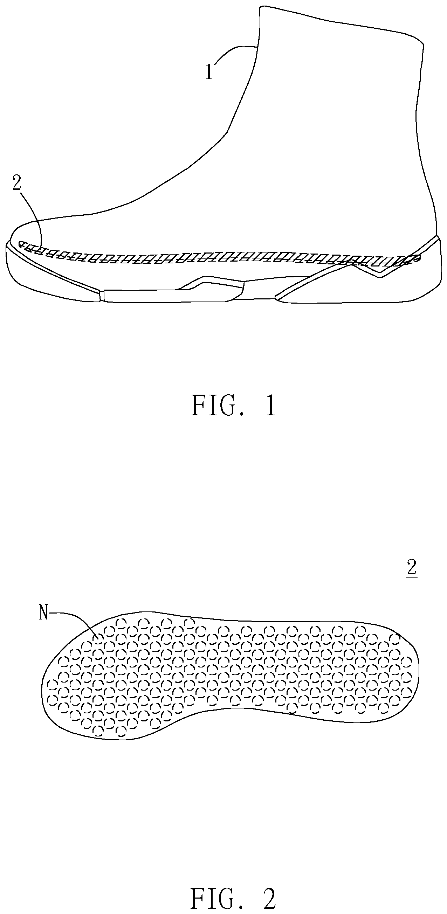

Assuming that the data processing apparatus is embedded in a capacitive pressure detection insole, the M original detection data received by the data processing apparatus can be M capacitance variations sensed by M capacitive sensing nodes of the capacitive pressure detection insole respectively. And, the M capacitance variations respectively correspond to M detection positions of the user's foot. Please refer to FIG. 1 and FIG. 2. FIG. 1 illustrates that the capacitive pressure detection insole 2 is disposed in a shoe 1; FIG. 2 illustrates that the capacitive pressure detection insole 2 has a plurality of capacitive sensing nodes N.

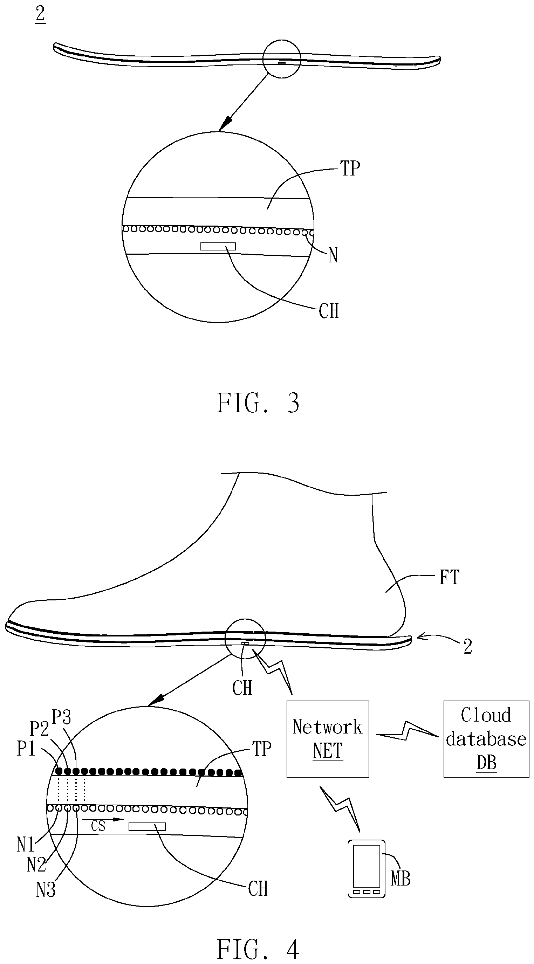

Next, please refer to FIG. 3 and FIG. 4. FIG. 3 is a schematic diagram showing that the capacitive pressure detection insole 2 has not been subjected to the pressure applied by the user's foot; FIG. 4 is a schematic diagram showing that the capacitive pressure detection insole 2 is subjected to the pressure applied by the user's foot FT.

As shown in FIG. 3 and FIG. 4, the capacitive pressure detection insole 2 includes a thermoplastic polyester elastomer (TPEE) layer TP, a plurality of capacitive sensing nodes N and an operating chip CH. The plurality of capacitive sensing nodes N is disposed under the thermoplastic polyester elastomer layer TP and the operating chip CH is embedded in the capacitive pressure detection insole 2.

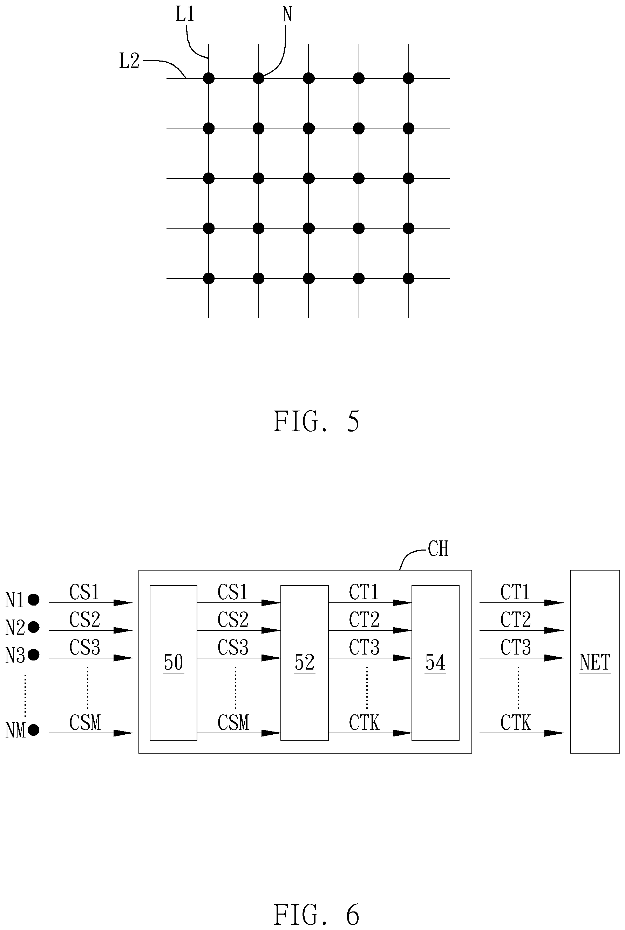

In practical applications, as shown in FIG. 5, the capacitive pressure detection insole 2 can be formed by twisting the capacitive yarn L1 and the conductive yarn L2 under the thermoplastic polyester elastomer layer TP, and the plurality of capacitance sensing node N can be located at the intersection of the capacitive yarn L1 and the conductive yarn L2, but not limited to this.

It should be noted that two electrically conductive coatings are formed on the surface of the capacitive yarn L1 and a capacitor is formed when the charges are distributed between the two electrically conductive coatings. When the capacitive pressure detection insole 2 has not been subjected to the pressure provided by the user's foot FT, the charge distribution between the two electrically conductive coatings is denser, that is, the charge density is higher; when the capacitive pressure detection insole 2 has been subjected to the pressure provided by the user's foot FT, the capacitive yarn L1 is crushed by the pressure, so that the charge distribution between the two electrically conductive coatings will become more dispersed. When the distance between the two electrically conductive coatings is constant, the capacitance is changed due to the decrease of the charge density, and the plurality of capacitance sensing nodes N located at the intersection of the capacitive yarn L1 and the conductive yarn L2 respectively sense a plurality of capacitance variations.

For example, as shown in FIG. 4, when the user's foot FT is stepped on the capacitive pressure detection insole 2, since the positions of the capacitive sensing nodes N1, N2, N3, ... of the capacitive pressure detection insole 2 correspond to the detection positions P1, P2, P3, ... of the foot FT respectively, the capacitive sensing nodes N1, N2, N3, ... of the capacitive pressure detection insole 2 can sense the capacitance variations corresponding to the detection positions P1, P2, P3, ... of the foot FT and transmit the capacitance variation CS to the operating chip CH. The operating chip CH can also be connected to the network NET and can be connected to the cloud database DB or the mobile communication device MB via the network NET.

Please refer to FIG. 6, the operating chip CH includes at least a receiving unit 50, a numerical transforming unit 52 and an output unit 54. The numerical transforming unit 52 is coupled between the receiving unit 50 and the output unit 54. When the receiving unit 50 receives M capacitance variations CS1∼CSM from the M capacitive sensing nodes N1∼NM, the receiving unit 50 transmits the M capacitance variations CS1∼CSM to the numerical transforming unit 52, and the numerical transforming unit 52 performs a numerical transforming process on the M capacitance variations CS1∼CSM by using a numerical transforming mechanism to generate K numerically transformed data CT1∼CTK, where M and K are both positive integers and K is less than M, resulting in that the amount of the K numerically transformed data generated by the numerical transforming unit 52 will be smaller than the amount of the M capacitance variations received by the numerical transforming unit 52, so as to achieve the effect of reducing the amount of data.

It should be noted that the numerical transforming mechanism adopted by the numerical transforming unit 52 can be a Fourier transform mechanism or a Laplace transform mechanism, but not limited to this. Next, the Fourier transform and Laplace transform will be explained separately:
Fourier transform is a linear integral transform, often used in the fields of physics and engineering to transform signals between time domain (or spatial domain) and frequency domain. For example, in signal processing, a typical use of Fourier transform is to decompose a signal into an amplitude component and a frequency component. Since the basic idea was first proposed systematically by the French scholar Joseph Fourier, it was named after his name to commemorate.

Laplace transform is a linear integral transform commonly used in applied mathematics to convert a function with an exponent real number (greater than or equal to 0) into a function with a complex argument. Because French astronomer and mathematician Pierre-Simon Laplace first used it in the study of probability theory, it was named after his name to commemorate.

Laplace transform is related to Fourier transform, but the difference is that Fourier transform represents a function or signal as a superposition of many sine waves, while Laplace transform represents a function as the superposition of many matrices. In physics and engineering, Laplace transform is often used to analyze linear time-invariant systems and convert between time and frequency domains, where both input and output are functions of time in the time domain (the unit is in seconds), and the input and the output in the frequency domain are functions of the complex angular frequency (the unit is in radians/second).

For example, if M is 1000, that is, the numerical transforming unit 52 receives 1000 capacitance variations CS1∼CS1000 corresponding to 1000 detection positions P1∼P1000 of the foot FT respectively, and the numerical transforming unit 52 can perform a sampling process on the 1000 capacitance variations CS1∼CS1000 by Fourier transform mechanism or Laplace transform mechanism, and a capacitance variation is sampled every 10 detection positions to generate 100 numerical transformed data CT1∼CT100 (i.e., K is 100). Since the amount of numerical transformed data is only 1/10 of the amount of original data, the total amount of data can be effectively reduced, so that the operating chip CH can perform the storing and processing procedure.

In addition, the operating chip CH can also reduce the amount of data by screening mechanism. For example, when the operating chip CH receives 1000 capacitance variations CS1∼CS1000 corresponding to 1000 detection positions P1∼P1000 of the foot FT respectively (that is, M is 1000), the operating chip CH can compare the capacitance variations CS1∼CS1000 with a preset value. If only 200 capacitance variations among the 1000 capacitance variations CS1∼CS1000 are larger than the preset value, it means that the other 800 capacitance variations among the 1000 capacitance variations CS1∼CS1000 are relatively small, which should be negligible. The operating chip CH will generate the numerical transformed data CT1∼CT200 according to the 200 capacitance variations greater than the preset value (that is, K is 200).

It should be noted that the advantage of this method is that the amount of data can be effectively reduced, and the unretained capacitance variations are relatively small, that is, the pressure distribution of the detection position of the foot FT corresponding the unretained capacitance variations has no obvious changes, so it can be negligible and will not affect the subsequent determination of the motion physiological status information of the foot.

After the numerical transforming unit generates the K numerical transformed data CT1∼CTK, the operating chip CH can store K numerical transformed data CT1∼CTK or analyze to determine the motion physiological status information of the user's foot according to the K numerical transformed data CT1∼CTK.

It should be noted that, after the numerical transforming unit 52 generates the numerical transformed data, in the subsequent applications, the numerical transformed data can be inversely transformed into the original detection data and then processed, or the lookup table is used to compare the original data corresponding to the K numerical transformed data CT1∼CTK respectively without specific limitations.

In practical applications, as shown in FIG. 6, the operating chip CH can also be connected to the network NET through the output unit 54 and output the K numerical transformed data CT1∼CTK to the network NET. As shown in FIG. 4, the operating chip CH can be connected to the cloud database DB through the network NET, and the operating chip CH can upload the pressure distribution information corresponding to the detection positions P1, P2, P3, ... of the foot FT and/or the data such as the motion physiological status information of the user's foot FT to the cloud database DB for reference for subsequent application.

For example, the insole manufacturer can obtain the motion physiological status information of the foot FT of the user A through the cloud database DB and determine the user's foot problem according to the motion physiological status information. Then, the insole manufacturer can customize a customized insole for the user A and set it in the shoe. When the user A wears a shoe with a customized correction insole and walks for a period of time, the user's foot problem should be significantly improved.

In addition, the user A can also operate the application (APP) on the mobile communication device (such as a smart phone) MB to connect to the operating chip CH or the cloud database DB through the network NET, so as to obtain the motion physiological status information of the foot FT of the user A at any time.

Another embodiment of the invention is a data amount reducing method, which can include the following steps: providing M original detection data, where M is a positive integer; and performing a numerical transforming process on the M original detection data to generate K numerical transformed data, wherein K is a positive integer smaller than M, so that an amount of the K numerical transformed data is smaller than an amount of the M original detection data.

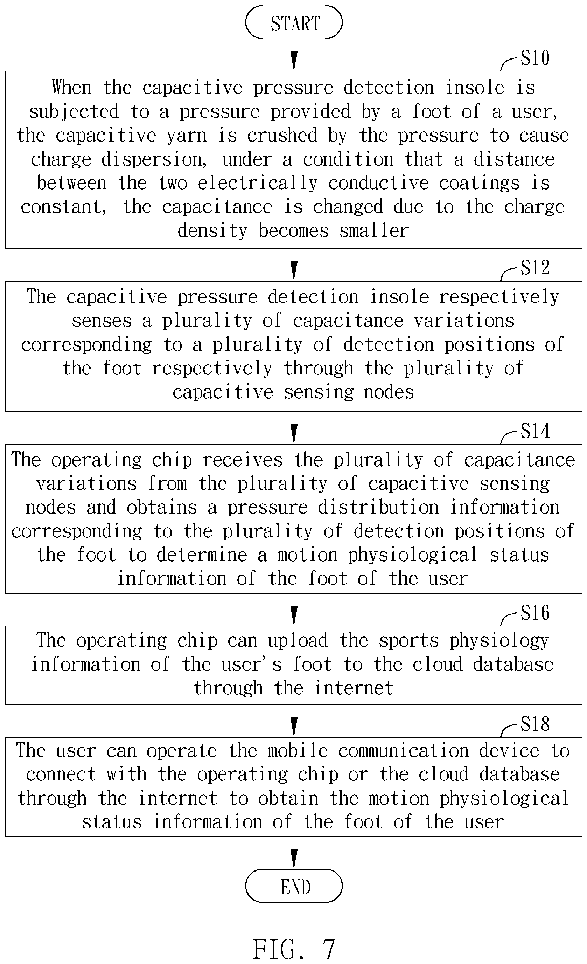

Please refer to FIG. 7. FIG. 7 is a flow chart showing the method for reducing the amount of data applied to a capacitive pressure detection insole in the invention. As shown in FIG. 7, the method can include the following steps:
Step S10: when the capacitive pressure detection insole is subjected to the pressure provided by the user's foot, two electrically conductive coatings are formed on the surface of the capacitive yarn and the capacitive yarn is crushed by the pressure to cause charge dispersion, in the case where the distance between the electrically conductive coatings is constant, the capacitance is changed due to the decrease in charge density.

Step S12: The capacitive pressure detection insole senses capacitance variations corresponding to the detection positions of the foot through the capacitive sensing nodes.

Step S14: The operating chip receives the capacitance variations from the capacitive sensing nodes and reduces the amount of data through a screening conversion mechanism to determine the motion physiological status information of the user's foot.

It should be noted that the operating chip can adopt a numerical transform program such as Fourier transform or Laplace transform, or can perform screening by comparing with a preset value to achieve the effect of reducing the amount of data, but not limited to this.

Step S16: The operating chip can upload the motion physiological condition information of the user's foot to the cloud database through the network.

Step S18: The user can operate the mobile communication device to connect to the operating chip or the cloud database through the network to obtain the motion physiological status information of the user's foot.

In one embodiment, the method of reducing the amount of data of the invention establishes pressure on a plurality of grid points on the insole through the pressure sensing yarn. When the consumer is in the motion process, these sensing points will sense the pressure on the insole as a function of time, and then record a function of the pressure versus time by sampling.

Assuming that there are 10,000 different pressure sensing points on the insole, each sensing point is sampled every 0.1 seconds and continuously sampled for 8 hours, the pressure measurement value of the insole sampled within 8 hours is 10,000 x. 10 x 60 x 60 x 8 = 2,880,000,000, or 2.8 billion data. This number may seem quite amazing on the surface, but it can be greatly simplified through an orderly model and recorded in a simple model.

In this embodiment, the invention provides useful information obtained from the insole which includes at least the following items:
(1) Four modes of standing/sitting, slow walking/fast walking, jogging/running, downhill/flat/uphill/distorted ground can be set. In each mode, when the user is in the early stage of learning, that is, the first stage model, the data lookup table is divided into four optional values of 2, 2, 2, 4, etc., and 32 different patterns are generated accordingly.
(2) For the standard actions (that is, the standard foot type, the standard motion, the standard height and weight (i.e., body type)) to test the sampling of the 32 modes for about 30 seconds. This means that each sampling takes approximately 10,000 x 10 x 30 = 3,000,000 (that is, 3 million sampling data). Overall, all 32 modes can establish approximately 96 million samples.
(3) Performing 10,000 Fast Fourier Transforms (FFT) on 300 sampling points for each 3 million points of data to find two important sets of values (A, F):
   F: peak frequency (finding the highest DB value, the frequencies of the first three different base frequency)
   A: amplitude of peak frequency (finding the amplitude DB value corresponding to these frequencies)
(4) If the peak frequency in (3) is an integer multiple of another peak frequency, the lowest frequency is called base frequency. The other integer multiple of frequency is the general frequency for the base frequency, which is used to determine the approximate waveform of each period of motion.
(5) A total of six sets of data have been obtained so far, and they can be divided into three main frequencies, each set of data has the following two data:
   Data I: (basic frequency, amplitude); (first general frequency multiple, amplitude); (second general frequency multiple, amplitude) ...
   Data II: For the 32 combined motion modes, after about 10 kinds of index type motion modes are set and the pressure versus time waveform is measure respectively, then a fast Fourier analysis (FFT) is done and compared with the waveform in the Data I to calculate the transformed frequency and the difference in amplitude of 32 groups of 10 indicator types of motion patterns.
      Delta [(basic frequency, amplitude); (first general frequency multiple, amplitude); (second general frequency multiple, amplitude)...]
(6) As to the two data in (5), Data I is the reference data and belongs to the data model of the standard motion pose. Data II is the offset generated by 10 error modes that deviate from the standard motion pose. Assuming that for a large number of 10,000 measurement points, 500 measurement points are offset from the standard motion pose, the following analysis can be performed for these 500 offset measurement points:
   (A) Find out which of the 32 modes are the most obvious deviations from the standard value on these 500 measurement points. Select three modes having the most obvious deviations from the 32 modes.
      Point (N) deviation 1 = Mode M1 (Delta F, Delta A, Delta F1, Delta A1, ....)
      Point (N) deviation 2 = Mode M2 (Delta F, Delta A, Delta F1, Delta A1, ....)
      Point (N) deviation 3 = Mode M3 (Delta F, Delta A, Delta F1, Delta A1, ....)

      It should be noted that the reason why the above-mentioned mode deviation can be determined is because the offset vector that the originally established standard error mode deviates from the delta value and the offset vector calculated according to the current data are oriented in the same direction. The Point (N) deviation is the ratio of the length of this offset vector to the length of the standard offset vector, which can represent the magnitude of the offset (proportional).
   (B) Record up to 3 general frequencies and each 2 data in the 3 modes (deviations) during each period of 30 seconds. Therefore, during every 30 seconds, there are up to 6 data recorded for each of the 500 points. So that, there are 3,000 data recorded during every 30 seconds.
   (C) These 500 abnormal points can be divided into a maximum of 10 regions, and the points with the highest degree of deviation in the specific mode are found in each region. In this way, only 10 points of data will be recorded. Therefore, the maximum amount of data recorded during every 30 seconds is only 60.
   (D) There are 2 x 60 x 8 = 960 30-second time periods in 8 hours, so the 8-hour motion record at these 10,000 measurement points can produce up to 60 x 960 x 2 = 115,200 Bytes data. Therefore, the total amount of data (that is, the IoT memory capacity) can be about 120 KB without a large amount of memory space, so the data amount M can be greatly simplified, and the operation and memory space can be saved.
(7) In order to simplify the complexity of collecting data of measurement points, the invention can also make 100 grid lines in the lateral and longitudinal directions of the insole. 25 measuring connectors are respectively arranged on the terminals of each grid line (400 terminals) to couple to the voltage signal sensors.

Compared to the prior art, the data processing apparatus and the data amount reducing method of the invention use the numerical transforming mechanism such as Fourier transform or Laplace transform or the screening mechanism comparing with a preset value to process the huge amount of received data to significantly reduce the amount of data, so that the data processing apparatus can store and process the numerical transformed data in real time, thus effectively avoiding the disadvantages of the prior art that the amount of data is too huge to be stored and processed.

With the example and explanations above, the features and spirits of the invention will be hopefully well described. Those skilled in the art will readily observe that numerous modifications and alterations of the device may be made while retaining the teaching of the invention. Accordingly, the above disclosure should be construed as limited only by the metes and bounds of the appended claims.

## Claims

1. A data processing apparatus, **characterized in that** the data processing apparatus comprises:
a receiving unit, configured to receive M original detection data, wherein M is a positive integer; and
a numerical transforming unit coupled to the receiving unit and configured to perform a numerical transforming process on the M original detection data to generate K numerical transformed data, wherein K is a positive integer smaller than M, so as to make the amount of the K numerical transformed data smaller than the amount of the M original detection data.

2. The data processing apparatus of claim 1, **characterized in that** the numerical transforming unit uses a numerical transforming mechanism to perform the numerical transforming process on the M original detection data.

3. The data processing apparatus of claim 2, **characterized in that** the numerical transforming mechanism is a Fourier transform mechanism or a Laplace transform mechanism.

4. The data processing apparatus of claim 1, **characterized in that** the data processing apparatus is a Bluetooth chip or an Internet of Thing (IoT) chip and can be connected to a cloud database or a mobile communication device through a network.

5. The data processing apparatus of claim 1, **characterized in that** the numerical transforming unit further compares the M original detection data with a preset value, and generates the K numerically transformed data according to the K original detection data that are greater than the preset value among the M original detection data.

6. The data processing apparatus of claim 1, **characterized in that** the M original detection data are M capacitance variations sensed by the M capacitive sensing nodes of a capacitive pressure detection insole, and the M capacitance variations respectively correspond to M detecting positions of a foot.

7. The data processing apparatus of claim 6, **characterized in that** one of the M capacitive sensing nodes of the capacitive pressure detection insole is disposed at an intersection of a capacitive yarn and a conductive yarn, and two electrically conductive coatings are formed on a surface of the capacitive yarn.

8. A data amount reducing method, **characterized in that** the data amount reducing method comprises steps of:
(a) providing M original detection data, wherein M is a positive integer; and
(b) performing a numerical transforming process on the M original detection data to generate K numerical transformed data, wherein K is a positive integer smaller than M, so that an amount of the K numerical transformed data can be smaller than an amount of the M original detection data.

9. The data amount reducing method of claim 8, **characterized in that** the step (b) uses a numerical transforming mechanism to perform the numerical transforming process on the M original detection data.

10. The data amount reducing method of claim 9, **characterized in that** the numerical transforming mechanism is a Fourier transform mechanism or a Laplace transform mechanism.

11. The data amount reducing method of claim 8, **characterized in that** the data amount reducing method further comprises steps of:
outputting the K numerical transformed data to a network; and
connecting to a cloud database or a mobile communication device through the network

12. The data amount reducing method of claim 8, **characterized in that** the data amount reducing method further comprises a step of:
comparing the M original detection data with a preset value, and generating the K numerically transformed data according to the K original detection data that are greater than the preset value among the M original detection data.

13. The data amount reducing method of claim 8, **characterized in that** the M original detection data are M capacitance variations sensed by the M capacitive sensing nodes of a capacitive pressure detection insole, and the M capacitance variations respectively correspond to M detecting positions of a foot.

14. The data amount reducing method of claim 12, **characterized in that** one of the M capacitive sensing nodes of the capacitive pressure detection insole is disposed at an intersection of a capacitive yarn and a conductive yarn, and two electrically conductive coatings are formed on a surface of the capacitive yarn.
